## Europäisches Patentamt
## European Patent Office
## Office européen des brevets

(19)

(11) Veröffentlichungsnummer: **0 047 796**
**B1**

(12) **EUROPÄISCHE PATENTSCHRIFT**

(45) Veröffentlichungstag der Patentschrift:
**14.12.83**

(21) Anmeldenummer: **80105529.4**

(22) Anmeldetag: **15.09.80**

(51) Int. Cl.³: **A 61 L 15/03,** A 61 L 15/01,
A 61 L 15/00, A 61 F 13/00,
D 04 H 1/54

(54) **Wundauflage aus hydrophoben Fasern.**

(43) Veröffentlichungstag der Anmeldung:
**24.03.82 Patentblatt 82/12**

(45) Bekanntmachung des Hinweises auf die Patenterteilung:
**14.12.83 Patentblatt 83/50**

(84) Benannte Vertragsstaaten:
**AT BE CH DE FR GB IT LI LU NL SE**

(56) Entgegenhaltungen:
**DE - A - 2 023 072**
**FR - A - 1 441 872**
**FR - A - 1 464 665**
**GB - A - 893 874**

(73) Patentinhaber: **Firma Carl Freudenberg, Höhnerweg 2,**
**D-6940 Weinheim/Bergstrasse (DE)**

(72) Erfinder: **Schmidt, Klaus, Dr., Haagackerweg 12,**
**D-6945 Hirschberg 1 (DE)**

(74) Vertreter: **Weissenfeld-Richters, Helga, Dr.,**
**Höhnerweg 2, D-6940 Weinheim/Bergstrasse (DE)**

ACTORUM AG

## Wundauflage aus hydrophoben Fasern

Die Erfindung betrifft eine Wundauflage aus hydrophoben Fasern, die untereinander physikalisch gebunden sind, vorzugsweise zur Verwendung als Abdeckschicht auf einem saugfähigen Wundverband.

Wundverbände haben den Zweck, eine offene Verletzung vor einer mechanischen Beschädigung oder einer Verunreinigung zu schützen, und gleichzeitig das aus der Wunde austretende Sekret aufzunehmen und in sich zu speichern, um dadurch die Wundheilung zu fördern.

Übliche Wundverbände bestehen aus einer Saugschicht aus Zellulose, die auf der der Wunde zugewandten Oberfläche mit einer Wundauflage aus hydrophoben Fasern abgedeckt ist, um zu verhindern, dass die Saugschicht während des Heilungsprozesses mit der Wunde verklebt.

Dabei muss zwangsläufig eine Beeinträchtigung der Saugwirkung der Saugschicht in Kauf genommen werden. Gemäss DE-OS 27 22 860 wurde deshalb bereits vorgeschlagen, die aus hydrophoben Fasern bestehende Wundauflage zusätzlich mit einem Netzmittel zu behandeln. In Hinblick auf die Notwendigkeit, diesbezüglich eine Substanz auszuwählen, die einerseits ausreichend fest an den glattflächigen, hydrophoben Fasern haftet, und die andererseits in physiologischer Hinsicht unbedenklich ist, verbleiben hinsichtlich der Auswahl nur sehr wenige Möglichkeiten. Eine grundsätzliche Verbesserung der bestehenden Situation konnte deshalb auch durch die Verwendung entsprechender Wundverbände nicht erzielt werden.

Der Erfindung liegt die Aufgabe zugrunde, eine Wundauflage insbesondere zur Verwendung als Abdeckschicht für saugfähige Wundverbände zu entwickeln, die bei einer guten Durchlässigkeit für vom Körper ausgeschiedene Flüssigkeiten zuverlässig ein Verkleben des Verbandes mit der Wunde verhindert, und die den Heilungsprozess positiv beeinflusst.

Diese Aufgabe wird bei einer Wundauflage der eingangs genannten Art dadurch gelöst, dass die Fasern wenigstens teilweise Mantelfasern mit einem offenporig geschäumten Mantel und einem ungeschäumten Faserkern aus demselben polymeren Werkstoff sind, und dass die Poren des Mantels bei einem Durchmesser von 0,01 bis 0,5 μm wenigstens 10% der Oberfläche des Mantels überdecken und dass sie mit einer die Wundheilung fördernden Substanz gefüllt sind.

Der Mantel und der Kern der Mantelfasern bestehen vorzugsweise aus Polycarbonat. Sie zeichnen sich in diesem Falle durch eine besonders grosse Weichheit und Verformbarkeit aus, wodurch sich ein entsprechender Wundverband besonders eng an die zu schützende Oberfläche einer offenen Wunde anschmiegt. Austretendes Körpersekret wird deshalb in allen Bereichen gleich günstig aufgenommen und in die hinter der Wundauflage angeordnete Speicherschicht eingelagert.

Andere polymere Werkstoffe, die sich vorteilhaft zur Herstellung der Wundauflage eignen, sind:
Polystyrol, Polyolefin und Polyurethan.

Die Wundauflage kann aus Fasern unterschiedlichen Durchmessers und unterschiedlicher Querschnittsform zusammengesetzt sein, was sich positiv auf die Oberflächenelastizität auswirkt. Im allgemeinen wird es jedoch bevorzugt, die Wundauflage aus Fasern gleichen Durchmessers und gleicher Querschnittsform herzustellen, vorzugsweise aus Fasern mit einem bandförmigen, flachen Profil. Solche Fasern können z.B. nach dem Verfahren nach DE-OS 20 23 072 erhalten werden. Insbesondere Fasern dieser Art eignen sich ausgezeichnet für die vollständige Abdeckung hervorstehender Faserenden oder -schlaufen aus der Oberfläche der abgedeckten Saugschicht, wobei die Gleichmässigkeit der Faserverteilung ein hervorzuhebender Vorteil ist. Vorstehende Faserbestandteile werden dadurch eingebunden und können nicht mehr in die Wunde einwachsen. Gleichzeitig ergibt sich eine verbesserte Kapillarwirkung zwischen den flach aufeinander abgelegten Mantelfasern, was zu einem verbesserten Feuchtigkeitstransport von der Wunde in das Innere der Saugschicht beiträgt.

Die Fasern der Wundauflage können durch eine Punktschweissung verbunden sein, derart, dass die Fasersubstanz aller Fasern in diskret verteilten Flächenbereichen vollkommen erschmolzen wird. Die einzelnen Fasern sind in die so erhaltenen fensterähnlichen Flächengebilde eingebunden und liegen in den Zwischenräumen vollkommen lose aufeinander auf. Eine solche Wundauflage zeichnet sich durch eine gute Geschmeidigkeit aus.

Eine verbesserte Saugfähigkeit wird demgegenüber erhalten, wenn die Fasern unter Vermeidung einer Zerstörung ihrer Identität fest verpresst werden, was zwischen glatten Stahlwalzen oder -platten geschehen kann. Die Temperatur soll unterhalb des Erweichungspunktes liegen. Die ausgezeichnete Festigkeit ist z.T. auf die grosse Oberflächenaktivität der Fasern zurückzuführen, z.T. auf die Porenstruktur des Mantels.

Die sehr gleichmässigen und runden Poren des geschäumten Mantels der Mantelfasern haben einen Durchmesser von 0,01 bis 0,5 μm, ein vorzugsweise einen Durchmesser von 0,05 bis 0,2 μm, wobei die Summe der Querschnitte der Poren wenigstens 10% und höchstens 95% der Oberfläche des Mantels überdeckt, bevorzugt nicht mehr als 70%. Die Porenverteilung und Grösse ist relativ gleichbleibend in allen Bereichen des Mantels, wobei die Gestalt bei einer Anordnung überwiegend senkrecht zur Oberfläche des Mantels als in etwa zylindrisch angegeben werden kann. Der flächenmässige Anteil des Mantels am Profil der Mantelfasern beträgt 40 bis 80%, vorzugsweise 60%. Das Porenvolumen eignet sich dadurch besonders gut für die Aufnahme verschiedenster, die Wundheilung fördernder Substanzen, die beispielsweise im Rahmen eines Imprägniervorganges eingebracht werden können. Besonders geeignet sind die folgenden Stoffe:

Antimikrobiologisch wirkende Stoffe wie Antibiotica, Sulfonamide, Antimykotica

Proteolytische Substanzen wie z.B. Trypsin Lokalanalgetische Substanzen wie z.B. Lidocain

Entzündungshemmende Substanzen wie z.B. Corticoide.

Aufgrund ihrer geringen Grösse sind die Poren relativ formstabil, was wesentlich dazu beiträgt, dass die eingelagerten Substanzen nur langsam gelöst und für die Wundheilung aktiviert werden. Die vorgeschlagene Wundauflage eignet sich aus diesem Grunde ausgezeichnet für die Herstellung von Langzeitverbänden, die beispielsweise für die Behandlung von Brandwunden benötigt werden.

Die Mantelfasern sind vorzugsweise Mikrofasern mit einem grössten Durchmesser von 1 bis 20 µm. Das Flächengewicht der Wundauflage ist abhängig von dem Grad der Unebenheiten der der Wunde zugewandten Seite der Saugschicht. Bei üblichen Saugschichten auf Zellwollbasis hat sich ein Wert von 5 bis 100 g/m² bewährt, wobei eine Grössenordnung zwischen 10 und 40 g/m² bevorzugt wird.

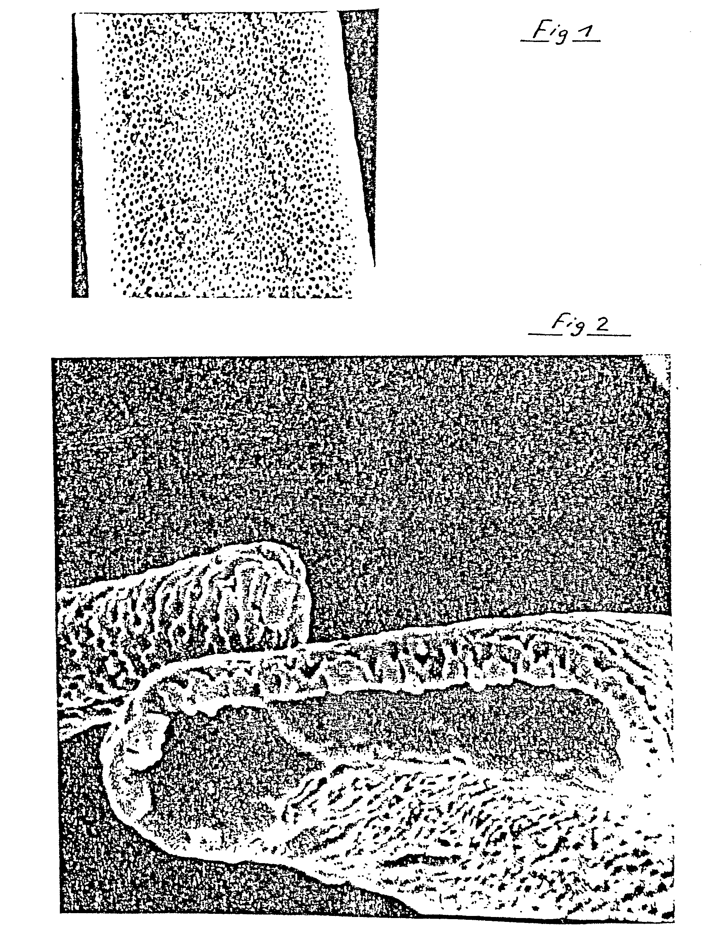

Die in der Anlage beigefügte Zeichnung nimmt in Figur 1 Bezug auf eine Mantelfaser der erfindungsgemäss verwandten Art, in Figur 2 auf dieselbe Faser in quergeschnittener Darstellung. Der Massstab beträgt im ersten Falle 7000:1, im letzten Falle 14000:1.

## Patentansprüche

1. Wundauflage aus hydrophoben Fasern, die untereinander physikalisch gebunden sind, vorzugsweise zur Verwendung als Abdeckschicht auf einem saugfähigen Wundverband, dadurch gekennzeichnet, dass die Fasern wenigstens teilweise Mantelfasern mit einem offenporig geschäumten Mantel und einem ungeschäumten Faserkern aus demselben polymeren Werkstoff sind, dass die Poren des Mantels bei einem Durchmesser von 0,01 bis 0,5 µm wenigstens 10% der Oberfläche des Mantels überdecken und dass sie mit einer die Wundheilung fördernden Substanz gefüllt sind.

2. Wundauflage nach Anspruch 1, dadurch gekennzeichnet, dass die Fasern aus Polycarbonat bestehen.

3. Wundauflage nach Anspruch 1 bis 2, dadurch gekennzeichnet, dass die die Wundheilung fördernden Stoffe eine antimikrobiologisch wirkende, eine proteolytische, lokalanalgetische oder entzündungshemmende Substanz ist.

4. Wundauflage nach Anspruch 1 bis 3, dadurch gekennzeichnet, dass die Poren einen Durchmesser von 0,05 bis 0,2 µm haben, und dass die Summe der Querschnitte der Poren nicht mehr als 95%, vorzugsweise nicht mehr als 70% der Oberfläche des Mantels überdeckt.

## Revendications

1. Garniture de pansement en fibres hydrophobes, liées physiquement l'une à l'autre, de préférence pour utilisation comme couche de couverture sur un pansement absorbant, caractérisée en e que les fibres sont au moins partiellement des fibres à enveloppe latérale, avec une enveloppe du type mousse à pores ouvertes et un noyau non du type mousse, en le même matériau polymère; en ce que les pores de l'enveloppe latérale, d'un diamètre de 0,01 à 0,5 microns s'étendent sur au moins 10% de la surface de l'enveloppe latérale; et en ce qu'elles sont remplies d'une substance qui favorise la guérison de la blessure.

2. Garniture de pansement selon la revendication 1, caractérisée en ce que les fibres sont en polycarbonate.

3. Garniture de pansement selon les revendications 1 à 2, caractérisée en ce que les produits qui favorisent la guérison de la blessure sont une substance à action antimicrobiologique, une substance protéolytique, localement analgésique ou une substance anti-inflammatoire.

4. Garniture de pansement selon les revendications 1 à 3, caractérisée en ce que les pores ont un diamètre de 0,05 à 0,2 microns; et en ce que la somme des sections des pores ne s'étend pas sur plus de 95%, de préférence ne s'étend pas sur plus de 70% de la surface de l'enveloppe latérale.

## Claims

1. A surgical compress consisting of hydrophobic fibers which are bonded among each other physically, preferably for use as cover layer on an absorptive wound dressing, characterized in that the fibers are at least partially fibers with a coat with an open pore foamed coat and an unfoamed fiber ocre of the same polymeric material, that the pores of the coat have a diameter of 0,01 to 0,5 µm covering at least 10% of the surface of the coat and that they are filled with a substance favoring the wound healing.

2. A surgical compress according to claim 1, characterized in that the fibers consist of polycarbonate.

3. A surgical compress according to claims 1 to 2, characterized in that the substances favoring the wound healing is an antimicro biologically acting, a proteolytic, local anaesthetic or an irritation preventing substance.

A surgical compress according to claims 1 to 3, characterized in that the pores have a diameter of 0,05 to 0,2 µm and that the sum of the cross sections of the pores does not cover more than 95%, preferably not more than 70% of the surface of the coat.

_Fig 1_

_Fig 2_